# EUROPEAN PATENT SPECIFICATION

(11) **EP 2 680 867 B1**
(45) Date of publication and mention of the grant of the patent: **15.08.2018**
(21) Application number: 12706618.1
(22) Date of filing: 05.03.2012
(51) Int. Cl.: A61K 35/76, C12N 7/04, C07K 14/705

(54) **ENHANCED TUMOR THERAPY BY TUMOR STEM CELL TARGETED ONCOLYTIC VIRUSES**
VERBESSERTE TUMORTHERAPIE DURCH AUF TUMORSTAMMZELLEN GEZIELTE ONKOLYTISCHE VIREN
THÉRAPIE ANTITUMORALE AMÉLIORÉE AVEC DES VIRUS ONCOLYTIQUES CIBLANT LES CELLULES SOUCHES TUMORALES

(30) Priority: 03.03.2011 EP 11156842
(43) Date of publication of application: 08.01.2014
(73) Proprietor: Bundesrepublik Deutschland, letztvertreten durch den Präsidenten des Paul-Ehrlich-Instituts Prof. Dr. Klaus Cichutek, 63225 Langen (DE)
(72) Inventor: BUCHHOLZ, Christian, 60594 Frankfurt (DE); BACH, Patricia, 63512 Hainburg (DE); ABEL, Tobias, 63225 Langen (DE)
(74) Representative: Grund, Martin
(86) International application number: PCT/EP2012/053728
(87) International publication number: WO 2012/117116

(56) References cited:
- WO-A2-03/093431
- WO-A2-2009/148488
- PENG KAH-WHYE ET AL: "Oncolytic measles viruses displaying a single-chain antibody against CD38, a myeloma cell marker.", BLOOD 1 APR 2003 LNKD- PUBMED:12433686, vol. 101, no. 7, 1 April 2003 (2003-04-01) , pages 2557-2562, XP002656476, ISSN: 0006-4971
- JOHAN SKOG ET AL: "Adenoviruses 16 and CV23 Efficiently Transduce Human Low-passage Brain Tumor and Cancer Stem Cells", MOLECULAR THERAPY, vol. 15, no. 12, 1 December 2007 (2007-12-01), pages 2140-2145, XP055004259, ISSN: 1525-0016, DOI: 10.1038/sj.mt.6300315
- MIZRAK D ET AL: "CD133: molecule of the moment.", THE JOURNAL OF PATHOLOGY JAN 2008 LNKD- PUBMED:18067118, vol. 214, no. 1, January 2008 (2008-01), pages 3-9, XP055004373, ISSN: 0022-3417
- DIAS J D ET AL: "Multimodal approach using oncolytic adenovirus, cetuximab, chemotherapy and radiotherapy in HNSCC low passage tumour cell cultures", EUROPEAN JOURNAL OF CANCER, PERGAMON PRESS, OXFORD, GB, vol. 46, no. 3, 1 February 2010 (2010-02-01), pages 625-635, XP026881616, ISSN: 0959-8049, DOI: 10.1016/J.EJCA.2009.11.005 [retrieved on 2009-12-16]
- Timothy P Cripe ET AL: "Targeting Cancer-initiating Cells With Oncolytic Viruses", Molecular Therapy, vol. 17, no. 10, 11 August 2009 (2009-08-11), pages 1677-1682, XP55200299, ISSN: 1525-0016, DOI: 10.1038/mt.2009.193
- SHENG-DONG HUANG ET AL: "Tumor Cells Positive and Negative for the Common Cancer Stem Cell Markers Are Capable of Initiating Tumor Growth and Generating Both Progenies", PLOS ONE, vol. 8, no. 1, 21 January 2013 (2013-01-21), page e54579, XP55200305, DOI: 10.1371/journal.pone.0054579
- YONATAN Y MAHLLER ET AL: "Neuroblastoma Cell Lines Contain Pluripotent Tumor Initiating Cells That Are Susceptible to a Targeted Oncolytic Virus", PLOS ONE, PUBLIC LIBRARY OF SCIENCE, US, vol. 4, no. 1, 1 January 2009 (2009-01-01) , pages e4235-1, XP008141769, ISSN: 1932-6203, DOI: 10.1371/JOURNAL.PONE.0004235 [retrieved on 2009-01-21]
- JAKUB NERADIL ET AL: "Nestin as a marker of cancer stem cells", CANCER SCIENCE, vol. 106, no. 7, 26 May 2015 (2015-05-26), pages 803-811, XP055353559, JP ISSN: 1347-9032, DOI: 10.1111/cas.12691
- DEY M ET AL: "Virotherapy against malignant glioma stem cells", CANCER LETTERS, NEW YORK, NY, US, vol. 289, no. 1, 1 March 2010 (2010-03-01) , pages 1-10, XP026897070, ISSN: 0304-3835, DOI: 10.1016/J.CANLET.2009.04.045 [retrieved on 2009-07-29]
- Timothy P Cripe ET AL: "Targeting Cancer-initiating Cells With Oncolytic Viruses", Molecular Therapy, vol. 17, no. 10, 11 August 2009 (2009-08-11), pages 1677-1682, XP055200299, ISSN: 1525-0016, DOI: 10.1038/mt.2009.193

## Description

### FIELD OF THE INVENTION

The invention relates to recombinant oncolytic viruses that target tumor stem cells and various uses of these recombinant viruses.

### BACKGROUND OF THE INVENTION

Tumors are thought to comprise a heterogeneous tumor cell population that differs in the degree of differentiation of the cells. A small fraction of the tumor cell population of a given tumor is made up of so-called tumor stem cells. It was shown that such tumor stem cells have a more pronounced ability to spread and/or generate new tumors than most of the further differentiated tumor cells of a given tumor. Such tumor stem cells can be identified by their surface markers, e.g. CD133.

The glycoprotein CD133 is primarily expressed on undifferentiated cells such as stem cells and precursor cells. The expression of CD133 has been shown for various stem cells such as hematopoietic stem cells, endothelial stem cells and neural stem cells and various tumors.

The fraction of CD133 positive tumor cells within a given tumor is quite low, however these cells are the prime source for new tumors, resistant to chemotherapy and feature a high regenerative potential.

Therefore, many types of cancer are still not curable and a need for new therapies exists.

Previous publications have proposed measles virus as cancer therapy. WO 03/093431 discloses a measles virus which is engineered to ablate SLAM-dependent entry and to target CD38-positive cells. Furthermore, Peng *et al.* disclose a measles virus which shows reduced binding to its natural receptors and specificity for CD38.

### SUMMARY OF THE INVENTION

The scope of the invention is defined by the appended claims. The present invention is based on the surprising finding that recombinant oncolytic viruses comprising a binding domain specific for a cell marker for a tumor stem cell can be utilized to specifically target/attack tumor stem cells comprised by a tumor, thereby inhibiting further growth and/or metastasizing of the tumor. Even more surprisingly, it has been found that such recombinantly modified oncolytic viruses exhibit a significantly increased oncolytic potential as compared to their non-modified original counterparts.
The present invention thus provides an improved treatment of various types of cancer, and even the treatment of such types that have as of yet been thought to be not curable, is possible.
The invention is directed to an oncolytic virus comprising a recombinant binding domain specific CD133+ which is a tumor stem cell marker.
The term **oncolytic virus** is meant to comprise any virus that infects/enters and lyses cancer cells. The ideal oncolytic virus efficiently kills a clinically relevant fraction of the patient's cancer cells by direct cytolysis with a minimal destruction of non-neoplastic tissue. Targeted tumor cell entry and specificity of replication are desirable. Furthermore, the virus should be safe and apathogenic when applied in patients. Oncolytic viruses derived from many different types of viruses have been described by Liu et al. (Liu et al., Nature Clinical Practice Oncology 4: (2) 101-117, 2007). Among these enveloped viruses such as herpes simplex virus (HSV), vaccinia virus (VV) and paramyxoviruses such as measles virus (MeV), Newcastle disease virus (NDV) or rhabdoviruses like vesicular stomatitis virus (VSV), are most prominent. Besides applying unmodified wildtype virus, genetic engineering can further improve safety and efficacy of oncolytic viruses. Engineering the envelope proteins can restrict virus infection to tumor cells and insertion of suicide genes can enhance therapeutic effects (Nakamura et al., Expert Opin. Bio. Ther. 4: (10): 1685-1692, 2004); Liu et al., Nature Clinical Practice Oncology 4: (2) 101-117, 2007).
The oncolytic virus of the invention is an enveloped virus derived from the *Paramyxoviridae* family, genus Morbillivirus, more preferably a measles virus (MeV) or a vaccine strain of MeV such as the Edmonston strain (MeV_{Edm}). MeV utilizes two envelope glycoproteins (the fusion protein (F) and the hemagglutinin protein (H)) to gain entry into the target cell. Protein F is a type I transmembrane protein, while protein H is a type II transmembrane domain, i.e. its amino-terminus is exposed directly to the cytoplasmic region. Both proteins thus comprise a transmembrane and a cytoplasmic region. One known function of the F protein is mediating the fusion of viral membranes with the cellular membranes of the host cell. Functions attributed to the H protein include recognizing the receptor on the target membrane and supporting F protein in its membrane fusion function. The direct and highly efficient membrane fusion at the cellular surface membrane is a particular property of measles virus and the morbilliviruses, thus distinguishing themselves from many other enveloped viruses that become endocytosed and will only fuse upon pH drop upon endocytosis. Both proteins are organized on the viral surface in a regular array of tightly packed spikes, H tetramers, and F trimers (Russell et al., Virology 199:160-168, 1994).

The Edmonston strain of MeV (MeV_{Edm}) uses a single protein as its main receptor, namely, the protein known to be the regulator of complement activation factor, CD46 (Gerlier et al., Trends Microbiol. 3:338-345, 1995). CD46 is expressed on all nucleated human cells. Most clinical isolates of measles virus, however, cannot effectively use CD46 as a receptor. Human SLAM (signaling lymphocyte-activation molecule; also known as CDw150) is a recently discovered membrane glycoprotein that is expressed on some T and B cells, and was also found to act as a cellular receptor for MeV, including the Edmonston strain (Tatsuo et al., Nature 406(6798):893-7, 2000). The precise biological functions and interactions of the MeV H and F proteins remain largely unclear.

The term **recombinant binding domain** is meant to comprise any domain of the virus that allows the virus to gain entry into the target cell and that is not present in the naturally occurring virus. The binding domain of the invention comprises a single-chain variable fragment (scFv). The binding domain comprises an scFv and is specific for the tumor stem cell marker CD133.

The term **specific for a tumor stem cell marker** is to be understood as the ability of the recombinant binding domain to interact with the tumor stem cell marker. Preferably, this interaction is a binding of the binding domain to said marker. More preferably, this binding is brought about by protein-protein interactions. Even more preferably, the binding domain acts as a ligand, while the tumor stem cell marker acts as a receptor. The concept of ligand and receptor interactions is well known to the skilled person. The term **cell marker** as used in the present invention, refers to a molecule present on the surface of a cell. Such molecules can be, inter alia, peptides or proteins that may comprise sugar chains or lipids, antigens, clusters of differentiation (CDs), antibodies or receptors. Since not all populations of cells express the same cell markers, a cell marker can thus be used to identify, select or isolate a given population of cells expressing a specific cell marker. As an example, CD4 is a cell marker expressed by T helper cells, regulatory T cells, and dendritic cells. Thus, T helper cells, regulatory T cells, and dendritic cells can be identified, selected or otherwise isolated, inter alia by a FACS cell sorter, by means of the CD4 cell marker. Likewise, CD133 is expressed on the surface of tumor stem cells. The cell marker of the present invention is a tumor stem cell marker, i.e. specific for tumor stem cells. The tumor stem cell marker is CD133.

The oncolytic virus of the invention has a decreased specificity for its original receptor(s) used for cell entry. In this embodiment the oncolytic virus of the present invention is preferably further modified in that the ligand(s) which are naturally used by the virus to gain entry to its host cell have a decreased specificity for their receptor(s). Preferably, the ligand(s) which are naturally used by the virus to gain entry to its host cell are modified to have no specificity or substantially no specificity for their receptor(s). Such a modification is preferably brought about by a mutation of the original ligands, more preferably by at least one point mutation within the ligand(s). The person skilled in the art will readily be able to introduce mutations as, for example, additions and deletions, into a given nucleic acid or amino acid sequence. Such known methodologies are, for example, disclosed in Sambrook et al. (1989). In an especially preferred embodiment in which the oncolytic virus is MeV, at least one point mutation is introduced into the H protein used for cell entry.

Mutation of the MeV H protein generally ablates productive interactions of the resulting Hmut protein with CD46 and SLAM, respectively. In one embodiment, this mutation is introduced by the point mutations Y481A and R533A of the MeV H protein. In another embodiment, the Hmut protein also includes the mutations S548L and/or F549S, which lead to a more complete ablation of residual infectivity via CD46. Also, the mutation of the residues V451 and Y529 ablates productive interaction with CD46 and SLAM. Alternative mutations for ablating/preventing interaction of the H protein with CD46 have been described above. All of these mutations, which are introduced into the H proteins in order to ablate the natural receptor usage, are located in the ectodomain of the MeV H protein. For preventing interaction of the H protein with SLAM one or more of the following residues may be replaced with any other amino acid, in particular, alanine: 1194, D530, Y553, T531, P554, F552, D505, D507 (See: Vongpunsawad et al. (2004) J Virol 78 (1) p. 302-313); Masse et al. (2004) J Virol 78 (17) p. 9051-9063).

The recombinant binding domain of the oncolytic virus is specific for CD133 (glioma, pancreatic adenocarcinoma, etc.). The CD133 cell marker is a five transmembrane domain glycoprotein (5-TM) that was initially shown to be expressed on primitive cell populations, including CD34⁺ hematopoietic stem and progenitor cells, and other primitive cells such as retina and retinoblastoma and developing epithelium. The CD133 antigen belongs to a newly characterized molecular family of 5-TM proteins. No natural ligand has yet been demonstrated for the CD133 molecule, and its precise function in hematopoietic tissue remains unknown.

The recombinant binding domain comprises an scFv. Preferably, said scFv is specific for CD133. In the most preferred embodiment the binding domain comprises an scFv derived from the hybridoma cell line deposited at ATCC as HB-12346. The generation of an scFv from a hybridoma cell line is a technique known to the skilled person and is exemplified in the appended examples (see Example 1).

In a further embodiment of the present invention the oncolytic virus further comprises at least one suicide gene. A suicide gene is a concept known to the skilled person. Generally it will, upon expression, cause a cell to be killed, preferably through apoptosis. In a further embodiment a suicide gene is used to make a tumor cell more sensitive to chemotherapy. Such an approach preferably involves a suicide gene that is coding for a toxic metabolite and/or an enzyme not found in the host of the tumor cell that can convert a harmless substance (pro-drug) into a toxic metabolite. A preferable suicide-transgene encodes for SuperCD, a fusion protein composed of yeast cytosine-deaminase and uracil-ribosyltranferase. When expressed from an oncolytic virus in tumor cells, it will convert the nontoxic prodrug 5-fluorocytosin, into the highly toxic compound 5-fluorouracil (Graepler et al., World J. Gastroenterol. 11: 6910-6919, 2005).

In another aspect the present invention is directed to the inventive oncolytic virus as a medicament. The oncolytic virus according to the invention is preferably comprised by a pharmaceutic composition.

Pharmaceutical compositions based on the oncolytic viruses of the present invention can be formulated in any conventional manner using one or more physiologically acceptable carriers or excipients. Thus, the oncolytic viruses of the present invention may be formulated for administration by, for example, injection, inhalation or insulation (either through the mouth or the nose) or by oral, buccal, parenteral or rectal administration.

The pharmaceutical compositions of the present invention can be formulated for a variety of modes of administration, including systemic, topical or localized administration. Techniques and formulations can be found in, for example, Remington's Pharmaceutical Sciences, Meade Publishing Co., Easton, Pa. For systemic administration, injection is preferred, including intramuscular, intravenous, intraperitoneal, and subcutaneous. For the purposes of injection, the pharmaceutical compositions of the present invention can be formulated in liquid solutions, preferably in physiologically compatible buffers, such as Hank's solution or Ringer's solution. In addition, the pharmaceutical compositions may be formulated in solid form and redissolved or suspended immediately prior to use. Lyophilized forms of the pharmaceutical composition are also suitable.

For oral administration, the pharmaceutical compositions of the present invention may take the form of, for example, tablets or capsules prepared by conventional means with pharmaceutically acceptable excipients such as binding agents (e.g. pregelatinized maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycolate); or wetting agents (e.g. sodium lauryl sulfate). The tablets can also be coated by methods well known in the art. Liquid preparations for oral administration may take the form of, for example, solutions, syrups or suspensions, or they may be presented as a dry product for constitution with water or another suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. ationd oil, oily esters, ethyl alcohol or fractionated vegetable oils); and preservatives (e.g. methyl or propyl-p-hydroxybenzoates or sorbic acid). The preparations can also contain buffer salts, flavoring, coloring and sweetening agents as appropriate.

The pharmaceutical compositions can be formulated for parenteral administration by injection, e.g. by bolus injection or continuous infusion. Formulations for injection can be presented in a unit dosage form, e.g. in ampoules or in multi-dose containers, with an optionally added preservative. The pharmaceutical compositions can further be formulated as suspensions, solutions or emulsions in oily or aqueous vehicles, and may contain other agents including suspending, stabilizing and/or dispersing agents.

Additionally, the pharmaceutical compositions can also be formulated as a depot preparation. These long acting formulations can be administered by implantation (e.g. subcutaneously or intramuscularly) or by intramuscular injection. Thus, for example, the compounds may be formulated with suitable polymeric or hydrophobic materials (e.g. as an emulsion in an acceptable oil) or ion exchange resins, or as sparingly soluble derivatives, for example, as a sparingly soluble salt. Other suitable delivery systems include microspheres, which offer the possibility of local noninvasive delivery of drugs over an extended period of time. This technology can include microspheres having a precapillary size, which can be injected via a coronary catheter into any selected part of an organ without causing inflammation or ischemia. The administered therapeutic is then slowly released from the microspheres and absorbed by the surrounding cells present in the selected tissue.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, bile salts, and fusidic acid derivatives. In addition, detergents may be used to facilitate permeation. Transmucosal administration can occur using nasal sprays or suppositories. For topical administration, the vector particles of the invention can be formulated into ointments, salves, gels, or creams as generally known in the art. A wash solution can also be used locally to treat an injury or inflammation in order to accelerate healing.

In a further embodiment the invention is directed to the oncolytic virus of the invention for the treatment or prevention of cancer, including, but not limited to, neoplasms, tumors, metastases, or any disease or disorder characterized by uncontrolled cell growth, and particularly multidrug resistant forms thereof. The cancer can be a multifocal tumor. Examples of types of cancer and proliferative disorders to be treated with the therapeutics of the invention include, but are not limited to, leukemia (e.g. myeloblastic, promyelocytic, myelomonocytic, monocytic, erythroleukemia, chronic myelocytic (granulocytic) leukemia, and chronic lymphocytic leukemia), lymphoma (e.g. Hodgkin's disease and non-Hodgkin's disease), fibrosarcoma, myxosarcoma, liposarcoma, chondrosarcoma, osteogenic sarcoma, angiosarcoma, endotheliosarcoma, Ewing's tumor, colon carcinoma, pancreatic cancer, breast cancer, ovarian cancer, prostate cancer, squamous cell carcinoma, basal cell carcinoma, adenocarcinoma, renal cell carcinoma, hepatoma, Wilms' tumor, cervical cancer, uterine cancer, testicular tumor, lung carcinoma, small cell lung carcinoma, bladder carcinoma, epithelial carcinoma, glioma, astrocytoma, oligodendroglioma, melanoma, neuroblastoma, retinoblastoma, dysplasia and hyperplasia. In a particular embodiment, therapeutic compounds of the invention are administered to patients having prostate cancer (e.g., prostatitis, benign prostatic hypertrophy, benign prostatic hyperplasia (BPH), prostatic paraganglioma, prostate adenocarcinoma, prostatic intraepithelial neoplasia, prostato-rectal fistulas, and atypical prostatic stromal lesions). In an especially preferred embodiment the medicaments of the present invention are used for the treatment of cancer, glioma, liver carcinoma and/or colon carcinoma. The treatment and/or prevention of cancer includes, but is not limited to, alleviating symptoms associated with cancer, the inhibition of the progression of cancer, the promotion of the regression of cancer, and the promotion of the immune response.

The pharmaceutical compositions of the present invention can be administered alone or in combination with other types of cancer treatment strategies (e.g., radiation therapy, chemotherapy, hormonal therapy, immunotherapy and anti-tumor agents). Examples of anti-tumor agents include, but are not limited to, cisplatin, ifosfamide, paclitaxel, taxanes, topoisomerase I inhibitors (e. g., CPT-11, topotecan, 9-AC, and GG-211), gemcitabine, vinorelbine, oxaliplatin, 5-fluorouracil (5-FU), leucovorin, vinorelbine, temodal, and taxo.

Additional embodiments of the present invention are described in the claims and the detailed description of the invention.

### DETAILED DESCRIPTION OF THE INVENTION

### BRIEF DESCRIPTION OF THE FIGURES

- **Fig. 1:**: Gives an overview over the cloning of MV-CD133, a recombinant tumor stem cell specific and oncolytic measles virus according to the invention.
- **Fig. 2:**: Shows the results of FACS and immuno-staining experiments to verify the tumor stem cell selectivity of MV-CD133.
- **Fig. 3:**: Shows the selectivity of MV-CD133 for CD133⁺ cells in cultures of mixed CD133⁺ and CD133⁻ cells.
- **Fig. 4:**: Shows the results of a FACS analysis of CD133 and GFP expression and analysis of the viability of cells infected with MV-CD133.
- **Fig. 5:**: Shows the oncolytic potential of MV-CD133 in immunodeficient (NOD/SCID) mice.
- **Fig. 6:**: Shows efficient elimination of multifocal tumors by MV-CD133
- **Figs. 7/8:**: Show the infection of Glioma tumorspheres by MV-CD133.
- **Fig. 9:**: Shows *in vivo* efficacy of MV-CD133 in a glioma model with MV-CD133-infected tumorspheres

### EXAMPLES

Envelope H protein of measles virus was modified to use human CD133 as a receptor for cell entry and to not bind to SLAM and CD46 anymore. The resulting recombinant virus is a tumor stem cell targeting oncolytic virus.

### EXAMPLE 1: Isolation and cloning of CD133 specific scFv and production of recombinant measles virus

For the generation of MV-CD133, RNA prepared from the AC141.7 antibody producing hybridoma HB-12346 generated as described in Yin et al., Blood, 90: 5002-5012.1997 was reverse-transcribed to amplify the IgG variable coding regions of heavy and light chains. A degenerated primer mix (Heavy Primer Mix, #27-1586-01; Light Primer Mix, #27-1583-01; GE Healthcare) was used for reverse transcription-PCR. The resulting PCR fragments were subcloned into the pJET1.2/blunt cloning vector (Fermentas) and then amplified to insert coding sequences for SfiI and NotI restriction sites and the (G₄S)₃-linker using CD133-VH and CD133-VL primer listed in Table 1. The resulting PCR fragments encoding the heavy or light chains were digested with TauI and SfiI, or TauI and NotI, respectively, and inserted by triple ligation into a SfiI and NotI-digested pCG-Hmut backbone resulting in pCG-Hmut-CD133scFv now encoding the cytoplasmic tail-truncated Hmut protein linked to the CD133-specific scFv (Funke et al., Mol.Ther. 16: 1427-1436, 2008; Anliker et al., Nat.Methods 7: 929-935, 2010)

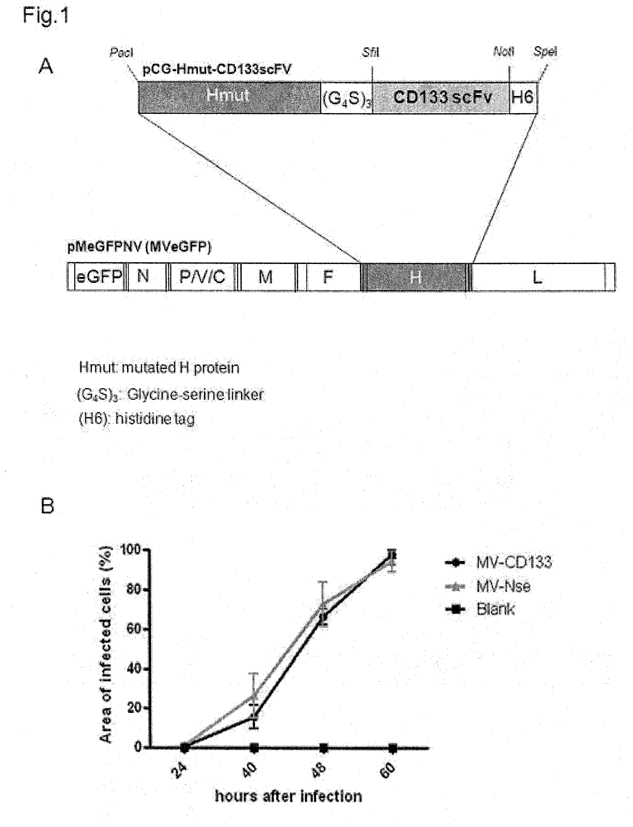

Next, the PacI/SpeI-digested fragment of pCG-Hmut-CD133scFv was inserted into the corresponding sites of pMeGFPNV (MVeGFP), which encodes a GFP-marked full-length infectious clone of the Edmonston lineage measles virus (Duprex et al., J. Virol. 73: 9568-9575, 1999) (Fig.1A). For the rescue of MV-CD133, the hexahistidine (His6) tagging and retargeting system was used as described previously (Nakamura et al., Nat.Biotechnol 23: 209-214, 2005) (Fig 1A). Virus stocks were generated upon infection of Vero-anti-His (anti-His) cells at a multiplicity of infection (MOI) of 0.03, and cell-associated viruses were harvested by multiple freeze-thaw cycles.

**Table 1: CD133-VH and CD133-VL Primer Sequences**

| **Primer** | **Sequence** |
|---|---|
| **CD133-VH forw1** | 5'-GGCCCAGCCGGCCATGGCCCAGGTCCAGCTGCAGGAGTCTGG-3' |
| **CD133-VH rev1** | 5'-GCCGCCACCTCCAGAGCCACCACCTCCCGAGGAGACGGTGACCGTGGTC-3' |
| **CD133-VL forw1** | 5'-GCGGCAGTGGTGGTGGAGGATCCGACATTGTCCTGACCCAGTCTCCA-3' |
| **CD133-VL rev1** | 5'-TGCGGCCGCCCGTTTTATTTCCAGCTTGGTCCC-3' |

To test the spreading capacity of retargeted MV-CD133 in tumor cells over time a virus spreading assay on HuH7 cells was performed. HuH7 cells were infected with the non-targeted MV-Nse or CD133 retargeted MVs at an MOI of 0.005 and cultured at 37°C for virus propagation. The number of GFP positive cells was determined at 24, 48, 72, 96 and 120 h after infection. MV-CD133 showed the same spreading kinetic as the parental MV-Nse strain (Fig.1B), demonstrating that the retargeted virus has no advantage in its spreading through HuH7 cells as compared to the parental vaccine strain.

### EXAMPLE 2: Verification of tumor stem cell selectivity

HuH7, Vero or HT1080 cells were tested for CD133 expression by flow cytometry applying anti-human CD133/1-PE antibody (clone AC133, Miltenyi). Appropriate isotype controls were used according to the manufacturer's instructions.

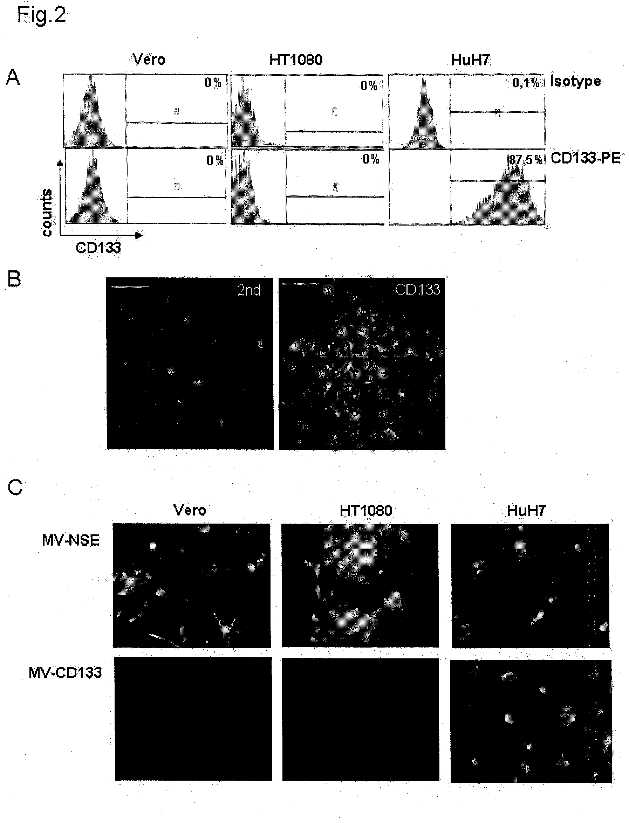

90% of HuH7 cells were CD133 positive whereas HT1080 and Vero cells were CD133 negative (Fig. 2A).

CD133 expression of HuH7 cells was confirmed by immunofluorescence staining using a mouse anti-human CD133 as primary antibody and a donkey anti-mouse IgG Cy3 (Dianova) secondary antibody (Fig. 2B).

HuH7, Vero and HT1080 cells (1×10⁴ cells/24-well plate) were incubated with MV-CD133 or MV-Nse at an MOI of 1 in Opti-MEM at 37 °C. At 72 hours after infection, cells were analysed by fluorescent microscopy. While MV-Nse infected all cell types, MV-CD133 infected only the CD133-positive HuH7 cells. Vero and HT1080 cells that are CD133-negative were not infected with MV-CD133 (Fig. 2C). Thus, MV-CD133 is highly selective for CD133-positive tumor stem cells.

### EXAMPLE 3: Specific infection of CD133⁺ cells by MV-CD133 in cultures of mixed CD133⁺ and CD133⁻ cells

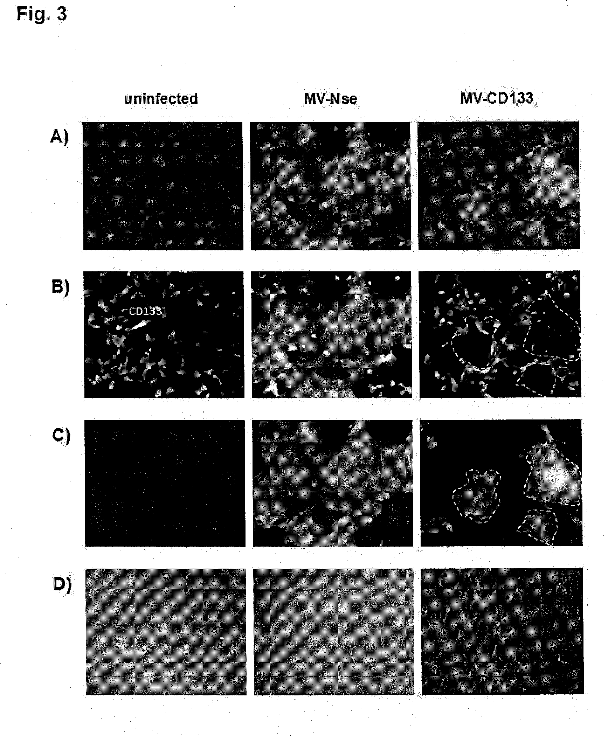

The target specificity of MV-CD133 was further determined in a cocultivation experiment of CD133⁺ cells (HT1080 cells genetically modified to express CD133) and CD133⁻ cells (wild-type HT1080 cells). To distinguish between both cell types CD133⁻ cells were genetically modified to express the red fluorescent protein (RFP). Thus, if these cells (HT1080) become infected with a GFP encoding measles virus they will turn yellow while the CD133⁺ cells (HT1080-CD133) will turn green. Both cell types were mixed in a 1:1 ratio and infected with MV-CD133 or MV-Nse at an MOI of 0.5, respectively. While MV-CD133 infected HT1080-CD133 cells only (Fig. 3A, right panel), MV-Nse infected both cell types resulting in yellow signals (Fig. 3A central panel). In Figure 3A of the microscopic pictures both colour channels are shown in overlay, in Fig. 3B-C the GFP (C) and RFP (B) channels are shown separately, to allow inspection of the Figure in absence of color . Arrows point to CD133⁻ cells. Dotted lines indicate areas of syncytia caused by MV-CD133 infection, which only appear in the GFP channel thus proving the specificity of MV-CD133 (Fig. 3C). Bright field pictures are shown in Fig. 3D. This shows that MV-CD133 is able to distinguish between CD133⁺ and CD133 cells even when these are in close contact as in tumor tissue.

### EXAMPLE 4: Viability of cells infected with MV-CD133

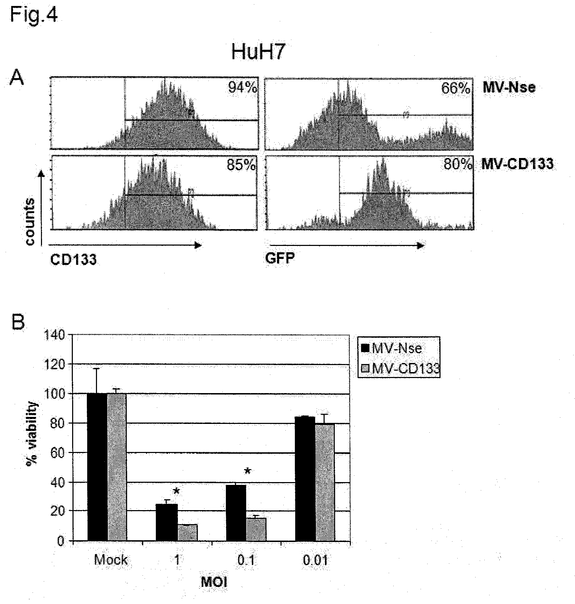

MV-CD133- and MV-Nse-infected HuH7 cells were analyzed for CD133 and GFP expression by FACS analysis. For this purpose, HuH7 cells were infected with an MOI of 1 and two days later the cells were trypsinized and stained for CD133 expression with an anti-human CD133/1-PE antibody and GFP positive cells were analyzed. With MV-CD133 approximately 80% of the cells had become GFP positive, with MV-Nse about 60% of the cells. MV-CD133 infection resulted in slight downregulation of CD133 cell surface expression (Fig. 4A).

To determine the viability of infected cells, the 3-(4,5-dimethylthiazol-2-yl)-2,5-diphenyltetrazolium bromide (Cell Proliferation Kit I (MTT); Roche, Indianapolis, IN) assay was used. Cells were grown in 96-well microtiter plates (1×10⁴ cells/well) in the recommended culture medium.

Infections were at MOI of 0 (mock), 0.01, 0.1, or 1. 96 hours post-infection cell viability was measured by dye absorbence as determined by an optical density measurement at 595 nm on an automated enzyme-linked immunosorbent assay reader. The viability of cells was calculated as the mean of quadruplicate optical density values, divided by the mean of quadruplicate optical density values of identically cultured cells in the absence of virus (which served as control cells) and expressed as a percentage of the control cells.

Compared to mock treated HuH7 cells, MV-CD133 infected HuH7 cells showed only 10% cell viability after infection at an MOI of 1. In case of MV-Nse infected HuH7 cells 20% of the cells were viable. Also at lower MOIs of 0.1 and 0.01 MV-CD133 killed HuH7 cells more efficiently than MV-Nse (Fig. 4B).

### EXAMPLE 5: Oncolytic potential of MV-CD133 in a NOD/SCID mice model.

To determine the oncolytic potential of MV-CD133, HuH7 cells (5 x10⁶ cells in 100 µl) were subcutaneously implanted into the right flank of 6 week old female nonobese diabetic/severe combined immunodeficient (NOD/SCID) mice (Jackson Labs).

After about 10 to 14 days, when the tumors measured 0.5-0.6 cm in diameter, mice received four (one per day) intratumoral injections of 1x10⁶ TCID50 in 100µl Opti-MEM of MV-Nse or MV-CD133 . Control animals (mock therapy groups) were injected with equal volumes of Opti-MEM containing no virus. Tumor growth was then followed over time.

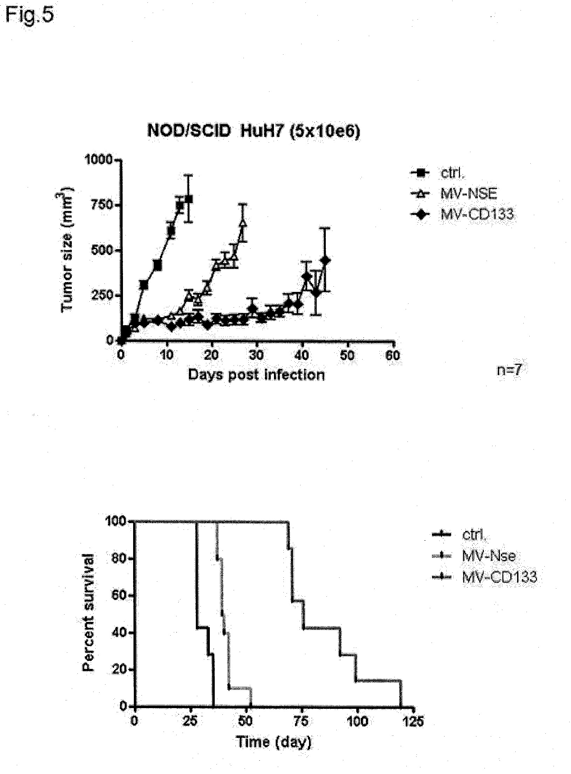

Mice that were treated with MV-CD133 showed a substantial reduction in tumor growth and a significantly prolonged survival period as compared to MV-Nse treated animals or control mice (Fig. 5).

### EXAMPLE 6: Efficient elimination of multifocal tumors by MV-CD133

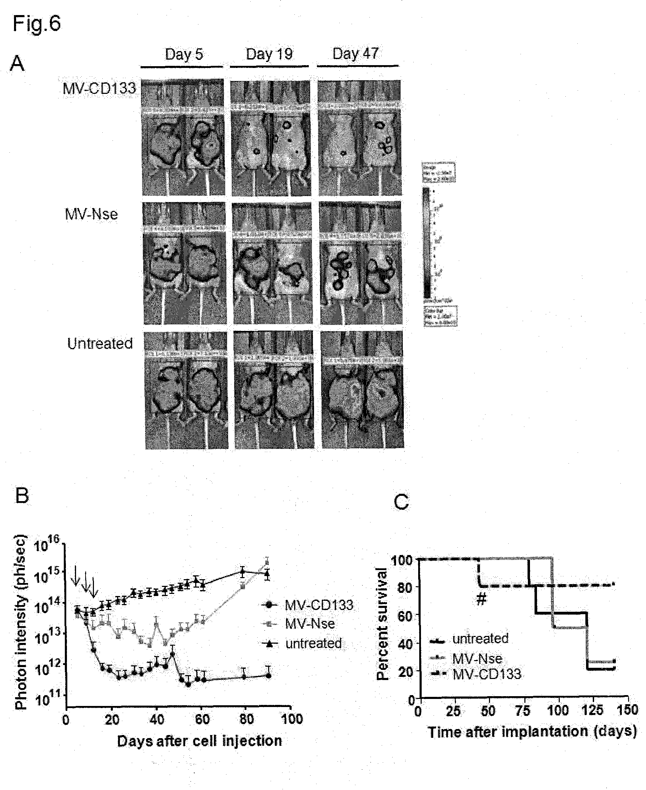

To assess the anti-tumoral potential of MV-CD133 in a multifocal tumor model with nodules growing at dispersed sites, HuH7 cells genetically modified to express luciferase (HuH7-luc cells) were injected intraperitoneally into athymic nude mice (n= 4-5) and tumor growth was monitored on day 5, 19 and 47 by *in vivo* imaging of the luciferase activity using the IVIS Spectrum imaging system. Seven days after cell administration multiple tumor foci were visible and mice were infected with MV-CD133 or MV-Nse via the intraperitoneal route. Control groups received cell culture medium only. Each animal received in total three virus injections every other day and tumor formation was monitored three times per week over a period of several weeks. Signal intensity was quantified as the mean of all detected counts within the region of interest after subtraction of background. Control mice showed enhanced tumor cell proliferation over time, whereas reduced tumor growth was observed in parental MV-Nse-treated animals on day 19 (Fig. 6A). The anti-tumoral effect was even more pronounced in mice treated with MV-CD133. Those animals were found to be tumor-free over a long time period (Fig. 6A).

Quantitative data for the luciferase bioluminescence intensities of tumors infected with MV-CD133 (circles), MV-Nse (squares) or control (triangle) are depicted in Fig.6B. Error bars represent mean 95% confidence intervals. Arrows depict the time points of virus injection. Animals treated with MV-CD133 showed regression of tumors after 3 weeks of the first treatment. Tumors injected with MV-Nse demonstrated a slight tumor reduction in the first weeks after treatment, but tumor regrowth occurred gradually from the 40th day onwards throughout the treatment period. In contrast to that, control treated animals showed progressive tumor growth over time until mice had to be sacrificed due to significant weight loss. Moreover, MV-CD133-treated mice showed a significantly longer survival compared to MV-Nse or control-treated animals (Fig.6C). One mouse in the MV-CD133 group died because of an accident (labeled by #).

In summary, MV-CD133 was able to substantially reduce tumor burden not only in a subcutaneous model but also in a multifocal tumor model. Most importantly, its oncolytic activity was significantly enhanced as compared to the non-targeted MV-Nse virus which is currently applied in clinical trials.

### EXAMPLE 7: Infection of glioma tumorspheres by MV-CD133

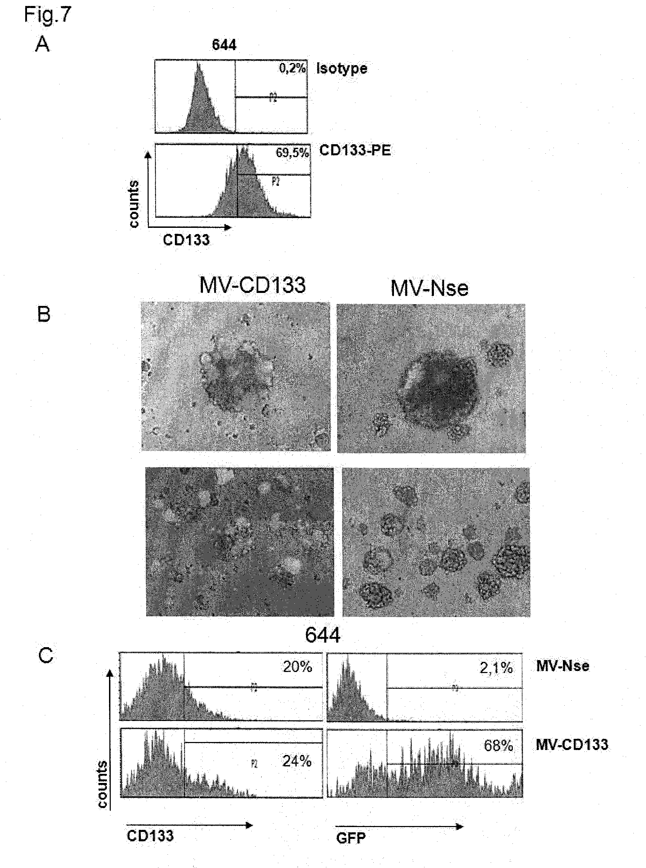

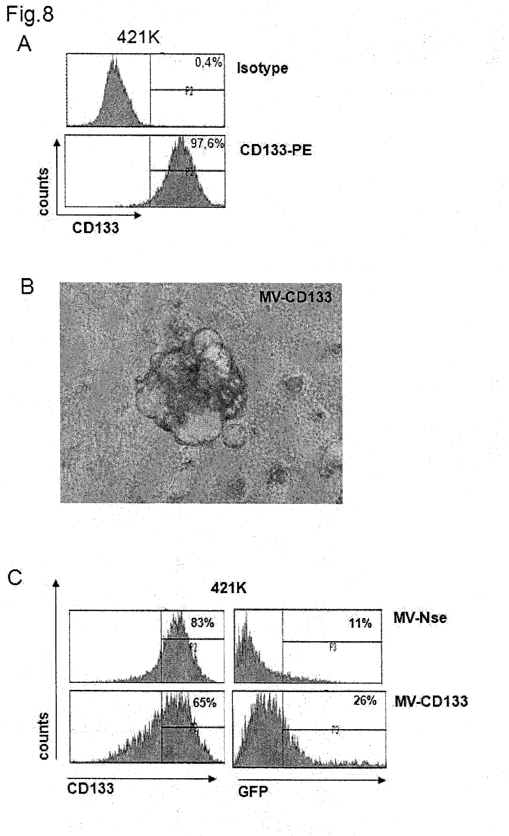

MV-CD133 was tested for infection of glioma tumorspheres from two different patients (644 or 421K) (Fig. 7 and 8). For this purpose, tumorspheres (1×10⁴ cells/24-well plate) were incubated with viruses (MV-CD133 or MV-Nse) at an MOI of 1 in Opti-MEM at 37 °C. At 48 to 72 hours after infection, cells were photographed by fluorescence microscope.

Both tumorsphere lines were CD133 positive (Fig. 7A and 8A) and were readily infected with MV-CD133 and MV-Nse.

In case of MV-CD133 infection resulted in strong syncitia formation. MV-Nse was also able to infect tumorspheres but to a much reduced level (Fig. 7B and 8B). GFP expression analysis of cells by microscopy correlated well with data obtained by FACS analysis.

Both tumorsphere lines showed high GFP expression levels after MV-CD133 infection and less GFP expression upon infection with MV-Nse (Fig. 7C and 8C).

### EXAMPLE 8: Oncolytic activity of MV-CD133 in an orthotopic model of primary glioma tumorspheres

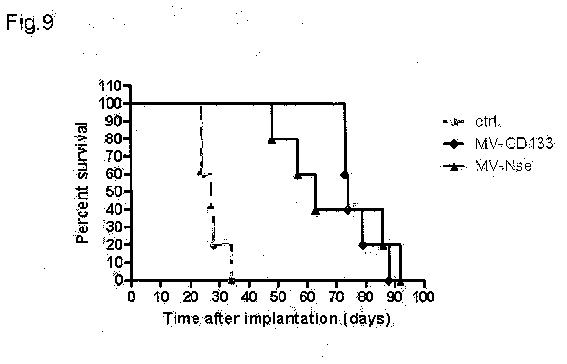

To assess the oncolytic activity of MV-CD133 in a setting close to the clinical situation of glioma patients, primary tumor cells were grown as tumorspheres. Upon infection with MV-CD133 or MV-Nse (MOI of 0.5) tumor cells were implanted into the right hemisphere of NOD/SCID mice (n=5) 16 h post infection. Survival of treated animals was followed over time. Mice that received control-treated tumor cells died within 30 days after implantation, whereas animals which had obtained MV-CD133 or MV-Nse-treated glioma cells survived 70 to 90 days (Fig. 9). Interestingly, some of the mice injected with MV-Nse-treated tumor cells died considerably earlier than mice injected with MV-CD133-treated tumor cells, but overall this difference in survival between both groups was not significant. However, also in this tumor model, MV-CD133 was at least as effective as non-targeted measles virus.

## Claims

1. An oncolytic virus comprising a recombinant binding domain specific for a tumor stem cell marker,
wherein the tumor stem cell marker is CD133,
wherein the virus has a decreased specificity for its original receptor(s) used for cell entry,
wherein the recombinant binding domain comprises a single-chain variable fragment (scFv), and
wherein the virus is from the Paramyxoviridae family, genus Morbillivirus.

2. The oncolytic virus according to claim 1, wherein the scFv is derived from hybridoma cell line ATCC HB-12346.

3. The oncolytic virus according to any of the preceding claims further comprising a suicide gene.

4. The oncolytic virus according to any of the preceding claims, wherein the virus is a measles virus (MeV).

5. The oncolytic virus according to any of the preceding claims for use as a medicament.

6. The oncolytic virus according to the use of claim 5 for the treatment or prevention of cancer, optionally wherein the cancer is a multifocal tumor.

7. The oncolytic virus according to the use of claim 6, wherein the cancer is glioma, liver carcinoma and/or colon carcinoma.

8. The oncolytic virus according to the use of claim 6 or 7 to be used in combination with other types of cancer treatment strategies.

## Patentansprüche

1. Ein onkolytischer Virus, umfassend eine rekombinante Binde-Domäne, die spezifisch für einen Tumor-Stammzellmarker ist,
wobei der Tumor-Stammzellmarker CD133 ist,
wobei der Virus eine verminderte Spezifizität für seine/n originalen Rezeptor/en, der/die für den Eintritt in die Zelle genutzt wird/werden, hat
wobei die rekombinante Binde-Domäne ein Single-chain variables Fragment (scFv) umfasst, und
wobei der Virus aus der Familie der Paramyxoviridae, Gattung Morbillivirus ist.

2. Der onkolytische Virus nach Anspruch 1, wobei das scFv aus der Hybridom Zelllinie ATCC HB-12346 gewonnen ist.

3. Der onkolytische Virus nach einem der vorherigen Ansprüche, der ferner ein Selbstmord-Gen umfasst.

4. Der onkolytische Virus nach einem der vorherigen Ansprüche, wobei der Virus ein Masernvirus (MeV) ist.

5. Der onkolytische Virus nach einem der vorherigen Ansprüche zur Verwendung als Medikament.

6. Der onkolytische Virus nach der Verwendung von Anspruch 5 zur Behandlung oder Prävention von Krebs, wobei gegebenenfalls der Krebs ein multifokaler Tumor ist.

7. Der onkolytische Virus nach der Verwendung von Anspruch 6, wobei der Krebs ein Gliom, Leberkarzinom und/oder Kolonkarzinom ist.

8. Der onkolytische Virus nach der Verwendung von Anspruch 6 oder 7, um zusammen mit anderen Arten von Krebsbehandlungsstrategien verwendet zu werden.

## Revendications

1. Virus oncolytique comprenant un domaine de liaison recombinant spécifique d'un marqueur de cellule souche tumorale,
dans lequel le marqueur de cellule souche tumorale est le CD133,
dans lequel le virus présente une spécificité diminuée pour son/ses récepteur(s) original/originaux utilisé(s) pour l'entrée dans la cellule,
dans lequel le domaine de liaison recombinant comprend un fragment variable monocaténaire (scFv), et
dans lequel le virus provient de la famille des Paramyxoviridés, du genre Morbillivirus.

2. Virus oncolytique selon la revendication 1, dans lequel le scFv est dérivé de la lignée cellulaire d'hybridome ATCC HB-12346.

3. Virus oncolytique selon l'une quelconque des revendications précédentes comprenant en outre un gène suicide.

4. Virus oncolytique selon l'une quelconque des revendications précédentes, dans lequel le virus est un virus de la rougeole (MeV).

5. Virus oncolytique selon l'une quelconque des revendications précédentes pour une utilisation en tant que médicament.

6. Virus oncolytique selon l'utilisation de la revendication 5 pour le traitement ou la prévention du cancer, éventuellement dans lequel le cancer est une tumeur multifocale.

7. Virus oncolytique selon l'utilisation de la revendication 6, dans lequel le cancer est un gliome, un carcinome du foie et/ou un carcinome du côlon.

8. Virus oncolytique selon l'utilisation de la revendication 6 ou 7 à utiliser en combinaison avec d'autres types de stratégies de traitement du cancer.


### SEQUENCE LISTING

<110> Bundesrepublik Deutschland, letztvertreten durch den Präsidenten des Paul-Ehrlich-Instituts, Prof. Dr. Klaus Cichutek BUCHHOLZ, Christian BACH, Patricia ABEL, Tobias
<120> Enhanced Tumor Therapy by Tumor Stem Cell Targeted Oncolytic Viruses
<130> 141-007P
<150> EP11156842.4
   <151> 2011-03-03
<160> 4
<170> PatentIn version 3.5
<210> 1
   <211> 42
   <212> DNA
   <213> Artificial Sequence
<220>
   <223> Forward Primer
<400> 1
   ggcccagccg gccatggccc aggtccagct gcaggagtct gg 42
<210> 2
   <211> 49
   <212> DNA
   <213> Artificial DNA sequence
<220>
   <223> Reverse primer
<400> 2
   gccgccacct ccagagccac cacctcccga ggagacggtg accgtggtc 49
<210> 3
   <211> 47
   <212> DNA
   <213> Artificial DNA sequence
<220>
   <223> Forward primer
<400> 3
   gcggcagtgg tggtggagga tccgacattg tcctgaccca gtctcca 47
<210> 4
   <211> 33
   <212> DNA
   <213> Artificial DNA sequence
<220>
   <223> Reverse primer
<400> 4
   tgcggccgcc cgttttattt ccagcttggt ccc 33